# EUROPEAN PATENT APPLICATION

(11) **EP 4 563 581 A1**
(43) Date of publication of application: **04.06.2025**
(21) Application number: 23845570.3
(22) Date of filing: 26.07.2023
(51) Int. Cl.: C07D 519/00, A01N 43/90, A01P 7/04, A01P 7/02

(54) **FUSED RING COMPOUND WITH SULFUR-CONTAINING SUBSTITUENT, PREPARATION METHOD, INSECTICIDE COMPOSITION, AND USE**

(30) Priority: 29.07.2022 CN 202210906545
(71) Applicant: Jiangsu Flag Chemical Industry Co., Ltd, Nanjing, Jiangsu 210000 (CN)
(72) Inventor: ZHANG, Pu, Nanjing, Jiangsu 210000 (CN); ZHAO, Wen, Nanjing, Jiangsu 210000 (CN); WANG, Fengyun, Nanjing, Jiangsu 210000 (CN); YAO, Kaicheng, Nanjing, Jiangsu 210000 (CN)
(74) Representative: Proi World Intellectual Property GmbH
(86) International application number: PCT/CN2023/109212
(87) International publication number: WO 2024/022362

(57) **Abstract**

The present invention belongs to the field of agricultural insecticides and particularly relates to a fused ring compound with a sulfur-containing substituent, a preparation method, an insecticide composition, and use. Compared with the prior art, the present invention has the beneficial effects that: the fused ring compound with a sulfur-containing substituent provided by the present invention has good prevention and treatment effect on many kinds of pests at a relatively low test concentration, providing a basis for research and development of new pesticides.

## Description

The present invention relates to the field of agricultural insecticides, and specifically relates to a sulfur-containing substituted fused ring compound, a preparation method, an insecticide composition, and its application.

Fused heterocyclic compounds with pesticidal action are known and described, for example, in WO2014/119672A1, WO2015/002211A1, WO2017/026384A1, WO2018/015289A1, and WO2016/091731A1. However, some active compounds known from the aforementioned literature have drawbacks, not only in terms of their narrow application range, but also in terms of their unsatisfactory insecticidal or acaricidal activity.

Now, new fused heterocyclic derivatives have been discovered, their advantages surpass those of known compounds, for example, better biological or environmental characteristics, more application methods, better insecticidal or acaricidal activity, and good compatibility with crop plants. Fused heterocyclic derivatives can be combined with other agents to enhance efficacy, especially in combating difficult-to-control insects.

The present invention provides a fused heterocyclic compound with sulfur substituents, as well as its preparation method and the intermediates used in its preparation, and its function in controlling pests, and its use as an acaricide and/or insecticide for controlling animal pests, especially arthropods, and particularly insects and arachnids.

The first objective of the present invention is to provide a fused ring compound with a sulfur-containing substituent of formula (I): 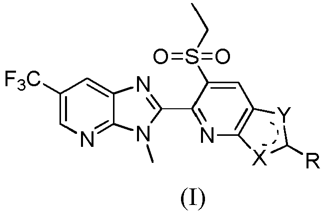 wherein,
X is selected from N, NR₁, O or S;
Y is selected from N or S; when Y is selected from N, X is selected from NR₁, O or S; when Y is selected from S, X is selected from N;
R₁ is selected from H, C_{1~6} alkyl, C_{1~6} halogenated alkyl, C_{3~6} cycloalkyl, C_{3~6} halogenated cycloalkyl;
R is selected from C_{1~6} alkyl, C_{1~6} substituted alkyl, C_{3~6} cycloalkyl, C_{3~6} substituted cycloalkyl, C_{2~6} epoxy, C₂₋₆ alkenyl, C₂₋₆ alkynyl, phenyl, substituted phenyl, C_{5~6} heterocycle or substituted heterocycle;
the substituent of substituted alkyl or substituted cycloalkyl is selected from halogen, C_{1~2} alkyl, C_{1~2} alkoxy, C_{1~2} halogenated alkoxy, C_{1~2} halogenated alkyl, cyano or ester group;
the substituted phenyl refers to phenyl substituted with 1, 2 or 3 substituents, the substituent is selected from halogen, C_{1~2} alkoxy, C_{1~2} halogenated alkoxy, cyano, C_{1~6} alkyl or C_{1~6} halogenated alkyl;
the heterocycle is selected from five-membered or six-membered monocyclic ring containing at least one non-carbon ring atom, including aromatic ring and non-aromatic hydrocarbon;
the substituted heterocycle refers to heterocycle substituted with 1, 2 or 3 substituents, the substituent of the substituted heterocycle is selected from halogen, C_{1~6} alkyl, C_{1~6} halogenated alkyl, C_{1~6} alkoxy or C_{1~6} alkylamide group.

Preferably, R₁ is selected from H, C_{1~6} alkyl, trifluoromethyl or cyclopropyl;
R is selected from C_{1~6} alkyl, C_{1~6} halogenated alkyl, C_{3~6} cycloalkyl, C₂₋₆ alkenyl, C₂₋₆ alkynyl, phenyl, substituted phenyl, C_{5~6} heterocycle or substituted heterocycle;
the substituted phenyl refers to phenyl substituted with 1, 2 or 3 substituents, the substituent is selected from halogen, C_{1~2} alkoxy, C_{1~2} halogenated alkoxy, cyano, C_{1~2} alkyl or C_{1~2} halogenated alkyl;
the heterocycle is selected from five-membered nitrogen-containing heterocyclic rings, five-membered sulfur-containing heterocyclic rings, five-membered oxygen-containing heterocyclic rings, six-membered nitrogen-containing heterocyclic rings, six-membered sulfur-containing heterocyclic rings or six-membered oxygen-containing heterocyclic rings
the substituent of the substituted heterocycle is selected from halogen, C_{1~2} alkyl, C_{1~2} halogenated alkyl, C_{1~2} alkoxy or C_{1~2} alkylamide group.

Further Preferably, R₁ is selected from C_{1~4} alkyl;
R is selected from C_{1~6} alkyl, C_{1~6} halogenated alkyl, C_{3~6} cycloalkyl, C₂₋₆ alkenyl, C₂₋₆ alkynyl, phenyl, substituted phenyl, C_{5~6} heterocycle or substituted heterocycle;
the substituted phenyl refers to phenyl substituted with 1, 2 or 3 substituents, the substituent is selected from halogen, methoxyl, cyano, ethoxyl, methyl, ethyl or trifluoromethyl;
the heterocycle is selected from piperazinyl, pyrrolidinyl, dioxanyl, morpholinyl, tetrahydrofuranyl, pyridyl, furanyl, imidazolyl, pyrimidinyl, oxazolyl, isoxazolyl, tetrazolyl, triazolyl, thiadiazolyl or thienyl;
the substituted heterocycle refers to heterocycle substituted with 1, 2 or 3 substituents, the substituent of the substituted heterocycle is selected from halogen, methyl, ethyl, methoxyl, trifluoromethyl or difluoromethyl.

Further Preferably, R₁ is selected from methyl;
R is selected from C_{1~6} alkyl, C_{1~6} halogenated alkyl, C_{3~6} cycloalkyl, C₂₋₆ alkenyl, C₂₋₆ alkynyl, phenyl, substituted phenyl, C_{5~6} heterocycle or substituted heterocycle;
the substituted phenyl refers to phenyl substituted with 1, 2 or 3 substituents, the substituent is selected from halogen, methoxyl, cyano, methyl or trifluoromethyl;
the heterocycle is selected from 2-thienyl, pyridyl, 2-chloropyridyl or 5-bromopyridyl;
the substituted heterocycle refers to heterocycle substituted with 1, 2 or 3 substituents, the substituent of the substituted heterocycle is selected from halogen, methyl, methoxyl or trifluoromethyl.

Further Preferably, R is selected from methyl, ethyl, trifluoromethyl, difluoromethyl, chloromethyl, cyclopropyl, heptafluoroisopropyl, isopropyl, n-propyl, isobutyl, tert-butyl, cyclobutyl, cyclopentyl, cyclohexyl, vinyl, ethynyl, propenyl, butenyl, propynyl, butynyl, phenyl, cyanocyclopropyl, 4-fluorophenyl, 2-fluorophenyl, 3-fluorophenyl, 2,4-difluorophenyl, 2-thienyl, 2-pyridyl, 2-chloropyridyl or 5-bromopyridyl.

Further Preferably, R₁ is selected from methyl; R is selected from cyclopropyl or trifluoromethyl.

In preferred embodiment of the present invention, compounds with chemical formulas (I-a), (I-b), (I-c), and (I-d) are provided:

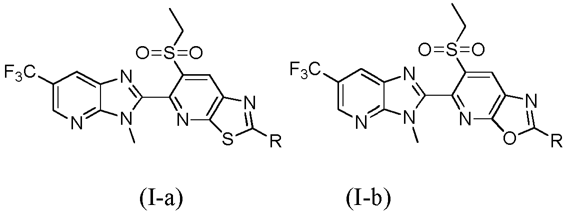

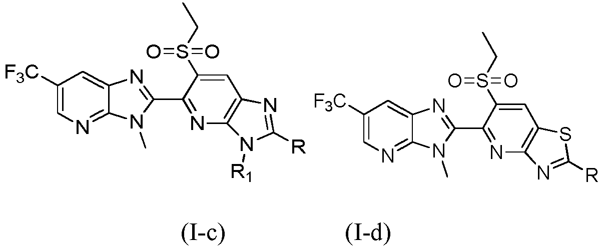

R₁ is selected from methyl; R is selected from methyl, ethyl, trifluoromethyl, difluoromethyl, chloromethyl, cyclopropyl, heptafluoroisopropyl, isopropyl, n-propyl, isobutyl, tert-butyl, cyclobutyl, cyclopentyl, cyclohexyl, vinyl, ethynyl, propenyl, butenyl, propynyl, butynyl, phenyl, cyanocyclopropyl, 4-fluorophenyl, 2-fluorophenyl, 3-fluorophenyl, 2,4-difluorophenyl, 2-thienyl, 2-pyridyl, 2-chloropyridyl or 5-bromopyridyl.

Further Preferably, R₁ is selected from methyl; R is selected from cyclopropyl or trifluoromethyl.

As the preferred specific compound structure of this invention, its substituents are shown in Table 1 (based on formula I):

**Table 1**

| Comp. No. | X | Y | R |
|---|---|---|---|
| I-1 | N-CH₃ | N | cyclopropyl |
| I-2 | N-CH₃ | N | -CH₃ |
| I-3 | N-CH₃ | N | -Ph |
| I-4 | N-CH₃ | N | -2-F-Ph |
| I-5 | N-CH₃ | N | -2,4-F-Ph |
| I-6 | N-CH₃ | N | -CH₂CH₃ |
| I-7 | N-CH₃ | N | isopropyl |
| I-8 | N-CH₃ | N | -CH₂CH₂CH₃ |
| I-9 | N-CH₃ | N | tert-butyl |
| I-10 | N-CH₃ | N | cyclobutyl |
| I-11 | N-CH₃ | N | Cyclopentyl |
| I-12 | N-CH₃ | N | cyclohexyl |
| I-13 | N-CH₃ | N | -3-F-Ph |
| I-14 | N-CH₃ | N | -4-F-Ph |
| I-15 | N-CH₃ | N | propenyl |
| I-16 | N-CH₃ | N | difluoromethyl |
| I-17 | N-CH₃ | N | cyclopropanecarbonitrile |
| I-18 | N-CH₃ | N | -2-Cl-Ph |
| I-19 | N-CH₃ | N | -2-Br-Ph |
| I-20 | N-CH₃ | N | -4-CH₃-Ph |
| I-21 | N-CH₃ | N | -2-CF₃-Ph |
| I-22 | N-CH₃ | N | -2-OCH₃-Ph |
| I-23 | N-CH₃ | N | -3-OCF₃-Ph |
| I-24 | N-CH₃ | N | -3,4-OCH₃-Ph |
| I-25 | N-CH₃ | N | ethynyl |
| I-26 | N-CH₃ | N | isobutyl |
| I-27 | N-CH₃ | N | -CH₂Cl |
| I-28 | N-CH₃ | N | trifluoromethyl |
| I-29 | N-CH₃ | N | ethenyl |
| I-30 | N-CH₃ | N | propynyl |
| I-31 | N-CH₃ | N | butenyl |
| I-32 | N-CH₃ | N | butynyl |
| I-33 | N-CH₃ | N | -2-thiophenyl |
| I-34 | N-CH₃ | N | -3-Py |
| I-35 | N-CH₃ | N | -2-Cl-Py |
| I-36 | N-CH₃ | N | -5-Br-Py |
| I-37 | O | N | cyclopropyl |
| I-38 | O | N | -CH₃ |
| I-39 | O | N | -Ph |
| I-40 | O | N | -2-F-Ph |
| I-41 | O | N | -2,4-F-Ph |
| I-42 | O | N | -CH₂CH₃ |
| I-43 | O | N | isopropyl |
| I-44 | O | N | -CH₂CH₂CH₃ |
| I-45 | O | N | tert-butyl |
| I-46 | O | N | cyclobutyl |
| I-47 | O | N | Cyclopentyl |
| I-48 | O | N | cyclohexyl |
| I-49 | O | N | -3-F-Ph |
| I-50 | O | N | -4-F-Ph |
| I-51 | O | N | propenyl |
| I-52 | O | N | difluoromethyl |
| I-53 | O | N | cyclopropanecarbonitrile |
| I-54 | O | N | -2-Cl-Ph |
| I-55 | O | N | -2-Br-Ph |
| I-56 | O | N | -2-CH₃-Ph |
| I-57 | O | N | -2-CF₃-Ph |
| I-58 | O | N | -2-OCH₃-Ph |
| I-59 | O | N | -3-OCF₃-Ph |
| I-60 | O | N | -3,4-OCH₃-Ph |
| I-61 | O | N | ethynyl |
| I-62 | O | N | isobutyl |
| I-63 | O | N | -CH₂Cl |
| I-64 | O | N | trifluoromethyl |
| I-65 | O | N | ethenyl |
| I-66 | O | N | propynyl |
| I-67 | O | N | butenyl |
| I-68 | O | N | butynyl |
| I-69 | O | N | -2-thiophenyl |
| I-70 | O | N | -3-Py |
| I-71 | O | N | -2-Cl-Py |
| I-72 | O | N | -5-Br-Py |
| I-73 | S | N | cyclopropyl |
| I-74 | S | N | -CH₃ |
| I-75 | S | N | -Ph |
| I-76 | S | N | -2-F-Ph |
| I-77 | S | N | -2,4-F-Ph |
| I-78 | S | N | -CH₂CH₃ |
| I-79 | S | N | isopropyl |
| I-80 | S | N | -CH₂CH₂CH₃ |
| I-81 | S | N | tert-butyl |
| I-82 | S | N | cyclobutyl |
| I-83 | S | N | Cyclopentyl |
| I-84 | S | N | cyclohexyl |
| I-85 | S | N | -3-F-Ph |
| I-86 | S | N | -4-F-Ph |
| I-87 | S | N | propenyl |
| I-88 | S | N | difluoromethyl |
| I-89 | S | N | cyclopropanecarbonitrile |
| I-90 | S | N | -2-Cl-Ph |
| I-91 | S | N | -2-Br-Ph |
| I-92 | S | N | -2-CH₃-Ph |
| I-93 | S | N | -2-CF₃-Ph |
| I-94 | S | N | -2-OCH₃-Ph |
| I-95 | S | N | -3-OCF₃-Ph |
| I-96 | S | N | -3,4-OCH₃-Ph |
| I-97 | S | N | ethynyl |
| I-98 | S | N | isobutyl |
| I-99 | S | N | -CH₂Cl |
| I-100 | S | N | trifluoromethyl |
| I-101 | S | N | ethenyl |
| I-102 | S | N | propynyl |
| I-103 | S | N | butenyl |
| I-104 | S | N | butynyl |
| I-105 | S | N | -2-thiophenyl |
| I-106 | S | N | -3-Py |
| I-107 | S | N | -2-Cl-Py |
| I-108 | S | N | -5-Br-Py |
| I-109 | N | S | cyclopropyl |
| I-110 | N | S | -CH₃ |
| I-111 | N | S | -Ph |
| I-112 | N | S | -2-F-Ph |
| I-113 | N | S | -2,4-F-Ph |
| I-114 | N | S | -CH₂CH₃ |
| I-115 | N | S | isopropyl |
| I-116 | N | S | -CH₂CH₂CH₃ |
| I-117 | N | S | tert-butyl |
| I-118 | N | S | cyclobutyl |
| I-119 | N | S | Cyclopentyl |
| I-120 | N | S | cyclohexyl |
| I-121 | N | S | -3-F-Ph |
| I-122 | N | S | -4-F-Ph |
| I-123 | N | S | propenyl |
| I-124 | N | S | difluoromethyl |
| I-125 | N | S | cyclopropanecarbonitrile |
| I-126 | N | S | -2-Cl-Ph |
| I-127 | N | S | -2-Br-Ph |
| I-128 | N | S | -4-CH₃-Ph |
| I-129 | N | S | -2-CF₃-Ph |
| I-130 | N | S | -2-OCH₃-Ph |
| I-131 | N | S | -3-OCF₃-Ph |
| I-132 | N | S | -3,4-OCH₃-Ph |
| I-133 | N | S | ethynyl |
| I-134 | N | S | isobutyl |
| I-135 | N | S | -CH₂Cl |
| I-136 | N | S | trifluoromethyl |
| I-137 | N | S | ethenyl |
| I-138 | N | S | propynyl |
| I-139 | N | S | butenyl |
| I-140 | N | S | butynyl |
| I-141 | N | S | -2-thiophenyl |
| I-142 | N | S | -3-Py |
| I-143 | N | S | -2-Cl-Py |
| I-144 | N | S | -5-Br-Py |

And the N-oxide or tautomer of the compounds in Table 1. "Ph" represents the phenyl, "Py" represents pyridyl group.

The second objective of the present invention is to provide a method for preparing the fused ring compound with a sulfur-containing substituent of formula (I-a), comprising: 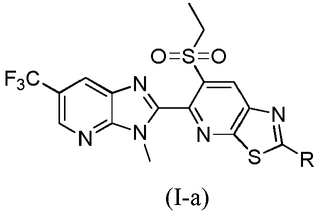
(1) stirring and refluxing a compound prepared by reacting a compound of formula (II) with a compound of formula (III) in acetic acid to give a compound of formula (IV); The process according to the invention for preparing compounds of formula (II) is carried out by methods known to those skilled in the art, or described for example in WO2014/119672A and WO2015/002211A; The compound of formula (III) can be prepared from commercially available 3-chloro-5-bromopyridine-2-carboxylic acid;
(2) contacting the compound of formula (IV) with ethanethiol under an alkaline condition to give a compound of formula (V);
(3) reacting the compound of formula (V) with an oxidant to give a compound of formula (VI);
(4) conducting a Buchwald coupling reaction of the compound of formula (VI) and 2,4-dimethoxybenzylamine to give a compound of formula (VII);
(5) reacting the compound of formula (VII) with trifluoroacetic acid to give a compound of formula (VIII);
(6) reacting the compound of formula (VIII) with a brominating reagent to give a compound of formula (IX);
(7) reacting the compound of formula (IX) with acyl chloride in the presence of an acid binding agent to give a compound of formula (X);
(8) reacting the compound of formula (X) with a Lawson's reagent to give a compound of formula (XI);
(9) reacting the compound of formula (XI) in the presence of a base to give the final target compound of formula (I-a); R and R₁ are as described previously, but do not limit the scope of the present invention;

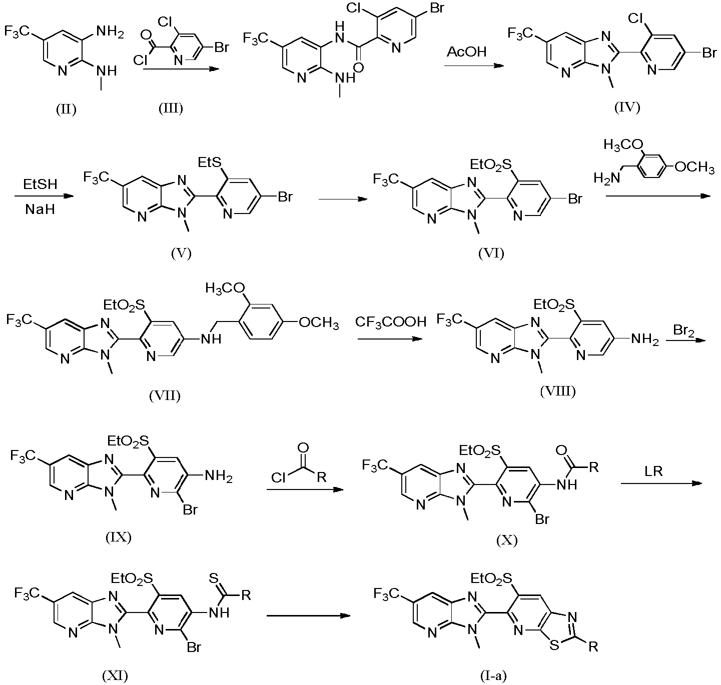

Preferably, in step (1), the solvent is selected from one or more of acetic acid, benzene and toluene, preferably acetic acid; the reaction temperature is 25~110°C, preferably 110°C; the volume of the solvent is 0.3~9 mL/mmol, preferably 0.4 mL/mmol, based on the molar amount of the compound represented by formula (II).

Preferably, in step (2), the solvent is selected from one or more of benzene, toluene, N,N-dimethylformamide and tetrahydrofuran, preferably tetrahydrofuran; the base is one or more of sodium hydride, sodium hydroxide and potassium hydroxide, preferably sodium hydride; The molar ratio of the reaction of formula (IV), ethanethiol and sodium hydride is 1:1~2:1~2, preferably 1:1:1.2; The reaction temperature is -10~80°C, preferably 0°C; The volume of the solvent is 0.3~9 mL/mmol, preferably 5 mL/mmol, based on the molar amount of the compound represented by formula (IV).

Preferably, in step (3), the solvent is one or more of dichloromethane, dichloroethane, methanol, ethanol, N,N-dimethylformamide and tetrahydrofuran, preferably dichloromethane; the oxidant is one or more of hydrogen peroxide and m-chloroperoxybenzoic acid, preferably hydrogen peroxide; the reaction temperature is 25~40°C, preferably 25°C; the volume of solvent is 0.3~9mL/mmol, preferably 5mL/mmol, based on the molar amount of the compound represented by formula (V).

Preferably, in step (4), the solvent is one or more of benzene, toluene and 1,4-dioxane, preferbly toluene; the reaction temperature is 25~150°C, preferably 120°C; the volume of solvent is 0.3~9mL/mmol, preferably 5mL/mmol, based on the molar amount of the compound represented by formula (VI).

Preferably, in step (5), the solvent is one or more of dichloromethane, dichloroethane, ethanol, methanol, acetonitrile and tetrahydrofuran, preferably dichloromethane; the reaction temperature is 25~80°C, preferably 25°C; the molar ratio of the compound represented by formula (VII) to trifluoroacetic acid is 1:1~10, preferably 1:2; the volume of the solvent is 3-9 mL/mmol, preferably 5 mL/mmol, based on the molar amount of the compound represented by formula (VII).

Preferably, in step (6), the solvent is one or more of dichloromethane, dichloroethane, tetrahydrofuran, toluene, 1,4-dioxane and acetic acid, preferably acetic acid; the bromination reagent is one or more of bromine and N-bromosuccinimide, preferably bromine; the reaction temperature is 25~80°C, preferably 80°C; the volume of the solvent is 3-9 mL/mmol, preferably 5 mL/mmol, based on the molar amount of the compound represented by formula (VIII).

Preferably, in step (7), the solvent is selected from one or more of dichloromethane, dichloroethane, tetrahydrofuran, *N,N*-dimethylformamide and acetonitrile, preferably tetrahydrofuran; the acid binding agent is one or more of triethylamine, potassium carbonate, sodium carbonate, potassium hydroxide, sodium hydroxide, pyridine, 4-dimethylaminopyridine, or sodium hydride, preferably triethylamine; the reaction temperature is 25~80°C, preferably 25°C; the volume of the solvent is 3-9 mL/mmol, preferably 5 mL/mmol, based on the molar amount of the compound represented by formula (IX).

Preferably, in step (8), the solvent is selected from one or more of dichloromethane, dichloroethane, tetrahydrofuran, N,N-dimethylformamide, or acetonitrile, preferably tetrahydrofuran; the molar ratio of the compound represented by formula (X) to Lawesson's reagent is 1:1~5, preferably 1:1.5; the reaction temperature is 25~80°C, preferably 80°C; the volume of the solvent is 3-9 mL/mmol, preferably 5 mL/mmol, based on the molar amount of the compound represented by formula (X).

Preferably, in step (9), the solvent is selected from one or more of 1,4-dioxane, dichloromethane, dichloroethane, tetrahydrofuran, N,N-dimethylformamide, or acetonitrile, preferably 1,4-dioxane; the base is one or more of sodium hydride, sodium carbonate, potassium carbonate, cesium carbonate, sodium hydroxide, and potassium hydroxide, preferably cesium carbonate; the reaction temperature is 25~100°C, preferably 80°C; the volume of the solvent is 3-9 mL/mmol, preferably 5 mL/mmol, based on the molar amount of the compound represented by formula (XI).

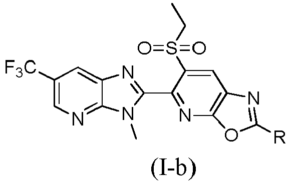

The similar method for preparing the compound of formula (I-b), comprising:
refluxing and stirring a compound of formula (X) in a solvent in the presence of CuI, a base and a catalyst to give the compound of formula (I-b);

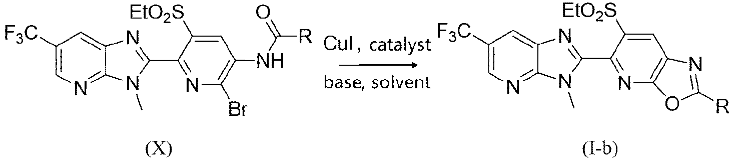

Preferably, the catalyst mentioned in the above steps is 1,10-phenanthroline; the base is one or more of sodium carbonate, potassium carbonate, and cesium carbonate, preferably cesium carbonate; the solvent is one or more of tetrahydrofuran, dichloromethane, dichloroethane, and ethylene glycol dimethyl ether, preferably ethylene glycol dimethyl ether; the volume of the solvent is 3-9 mL/mmol, preferably 5 mL/mmol, based on the molar amount of the compound represented by formula (X).

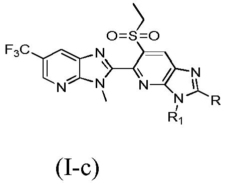

The similar method for preparing the compound of formula (I-b), comprising:
(1) Similar to the method for preparing compound (IV) in preparation (I-a), refluxing and stirring a compound of formula (II) in acetic acid to give a compound of formula (III);
(2) reacting the compound of formula (III) with ethanethiol in the presence of a base to give a compound of formula (IV);
(3) oxidizing the compound of formula (IV) to give a compound of formula (V);
(4) contacting the compound of formula (V) with a substituted amine in a solvent to give a compound of formula (VI);
(5) dissolving the compound of formula (VI) in concentrated sulfuric acid, and then adding dilute nitric acid dropwise at low temperature to give a compound of formula (VII);
(6) reducing the compound of formula (VII) to give a compound of formula (VIII);
(7) preparation method of formula (IX) is the same as that of formula (X) in (I-a);
(8) refluxing and stirring the compound of formula (IX) in acetic acid to give the compound of formula (I-c);

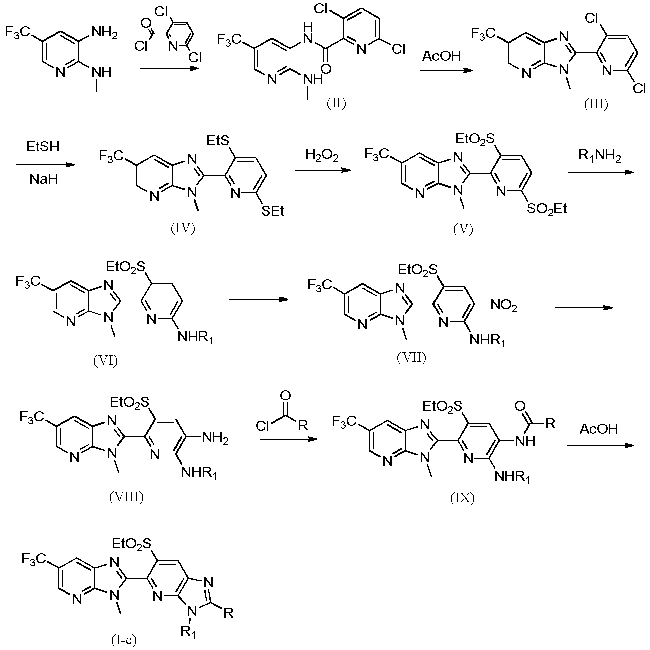

Preferably, in step (1), the solvent is selected from one or more of acetic acid, benzene, and toluene, preferably acetic acid; the reaction temperature is 25~110°C, preferably 110°C; the volume of the solvent is 0.3~9mL/mmol, preferably 0.4 mL/mmol, based on the molar amount of the compound represented by formula (II).

Preferably, in step (2), the solvent is one or more of benzene, toluene, *N,N-*dimethylformamide, or tetrahydrofuran, preferably tetrahydrofuran; the base is one or more of sodium hydride, sodium hydroxide, and potassium hydroxide, preferably sodium hydride; the molar ratio of the compound represented by formula (IV) to ethanethiol and sodium hydride in the reaction is 1:1~2:1~2, preferably 1:2:2; the reaction temperature is -10°C~80°C, preferably 0°C; the volume of the solvent is 0.3~9mL/mmol, preferably 5mL/mmol, based on the molar amount of the compound represented by formula (IV).

Preferably, in step (2), the solvent is one or more of dichloromethane, dichloroethane, methanol, ethanol, *N,N-*dimethylformamide, and tetrahydrofuran, preferably dichloromethane; the oxidant is one or more of hydrogen peroxide and m-chloroperoxybenzoic acid, preferably hydrogen peroxide. the reaction temperature is 25~40°C, preferably 25°C; the volume of the solvent is 0.3~9mL/mmol, preferably 5mL/mmol, based on the molar amount of the compound represented by formula (V).

Preferably, in step (4), the solvent is one or more of methanol, ethanol, *N,N-*dimethylformamide, or tetrahydrofuran, preferably tetrahydrofuran; the reaction temperature is 25~120°C, preferably 100°C.

Preferably, in step (5), the reaction temperature is -10~25°C, preferably 0°C.

Preferably, in step (6), the reducing agent is iron powder or palladium carbon, preferably palladium carbon.

Preferably, in step (7), the solvent is selected from one or more of dichloromethane, dichloroethane, tetrahydrofuran, *N,N*-dimethylformamide, or acetonitrile, preferably tetrahydrofuran; the acid binding agent is one or more of triethylamine, potassium carbonate, sodium carbonate, potassium hydroxide, sodium hydroxide, pyridine, 4-dimethylaminopyridine, or sodium hydride, preferably triethylamine; the reaction temperature is 0~80°C, preferably 25°C; the volume of the solvent is 3~9mL/mmol, preferably 5mL/mmol, based on the molar amount of the compound represented by formula (VIII).

Preferably, in step (8), the reaction temperature is 90~150°C, preferably 110°C.

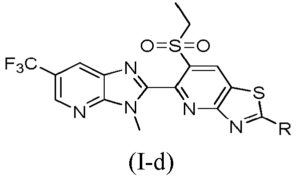

The similar method for preparing the compound of formula (I-d), comprising:
(1) contacting a compound of formula (V) obtained in (I-c) with ammonia solution in a solvent to give a compound of formula (X);
(2) brominating the compound of formula (X) in a solvent in the presence of a base to give a compound of formula (XI);
(3) preparation method of compound (XII) is the same as that of compound (IX) in (I-c);
(4) preparation method of compound (XIII) is the same as that of compound (XI) in (I-a);
(5) preparation method of formula (I-d) is the same as that of formula (I-a).

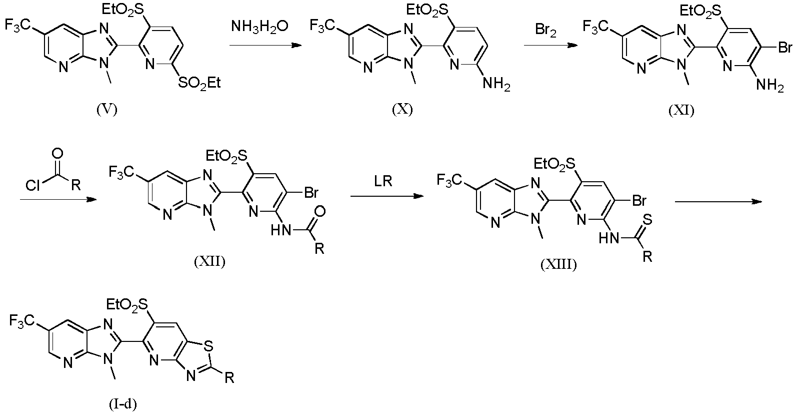

Preferably, in step (1), the solvent is one or more of methanol, ethanol, *N,N-*dimethylformamide, or tetrahydrofuran, preferably tetrahydrofuran; the reaction temperature is 25~120°C, preferably 100°C

Preferably, in step (1), the solvent is one or more of dichloromethane, dichloroethane, tetrahydrofuran, toluene, 1,4-dioxane, and acetic acid, preferably acetic acid; the bromination reagent is bromine or N-bromosuccinimide, preferably bromine; the base is sodium acetate or potassium acetate, preferably potassium acetate; the reaction temperature is 25~80°C, preferably 80°C; the volume of solvent is 3~9mL/mmol, preferably 5mL/mmol, based on the molar amount of the compound represented by formula (VIII).

Preferably, in step (3), the solvent is selected from one or more of dichloromethane, dichloroethane, tetrahydrofuran, *N,N*-dimethylformamide, or acetonitrile, preferably tetrahydrofuran; the acid binding agent is selected from one or more of triethylamine, potassium carbonate, sodium carbonate, potassium hydroxide, sodium hydroxide, pyridine, 4-dimethylaminopyridine, or sodium hydride, preferably triethylamine; the reaction temperature is 0~80°C, preferably 25°C; the volume of the solvent is 3~9mL/mmol, preferably 5mL/mmol, based on the molar amount of the compound represented by formula (XI).

Preferably, in step (4), the solvent is selected from one or more of dichloromethane, dichloroethane, tetrahydrofuran, *N,N*-dimethylformamide, or acetonitrile, preferably tetrahydrofuran; the molar ratio of the compound represented by formula (XII) to Lawesson's reagent is 1:1~5, preferably 1:1.5; the reaction temperature is 25~80°C, preferably 80°C; the volume of solvent is 3 to 9 mL/mmol, preferably 5 mL/mmol, based on the molar amount of the compound represented by formula (XII).

Preferably, in step (5), the solvent is selected from one or more of 1,4-dioxane, dichloromethane, dichloroethane, tetrahydrofuran, *N,N*-dimethylformamide, or acetonitrile, preferably 1,4-dioxane; the base is selected from one or more of sodium hydride, sodium carbonate, potassium carbonate, cesium carbonate, sodium hydroxide, or potassium hydroxide, preferably cesium carbonate; the reaction temperature is 25~120°C, preferably 80°C; the volume of the solvent is 3~9mL/mmol, preferably 5mL/mmol, based on the molar amount of the compound represented by formula (XIII).

The third aspect of the present invention provides the application of a fused ring compound with a sulfur-containing substituent of formula (I) in the preparation of insecticides.

Preferably, the insecticide is an insecticide for controlling lepidoptera and/or coleoptera; further, the pest can be one or more of *nilaparvata lugens, laodelphax striatellus, bemisia tabaci, plutella xylostella, mythmna separata, myzus persicae,* and *tetranychus cinnabarinus.*

The fourth aspect of the present invention provides an insecticide, which comprises a sulfur-containing substituent fused ring compound and an adjuvant, the concentration of the sulfur-containing substituent fused ring compound in the insecticide is 1~600 ppm; further, the concentration of the sulfur-containing substituent fused ring compound in the insecticide is 4~100 ppm.

In the event of a conflict between the Chinese nomenclature and the structural formula of the compound in this invention, the structural formula shall prevail.

Compared with existing technologies, the beneficial effects of the present invention lie in the fact that the fused ring compounds containing sulfur substituents provided by the invention exhibit good control effects on various pests at relatively low test concentrations, providing a basis for the development of new pesticides.

### Embodiment

The following describes the invention with examples, but it does not limit the invention. Within this field, simple substitutions or improvements made by skilled personnel to the invention fall within the technical solution protected by the invention.

### Example 1:

### Synthesis of Compound I-1

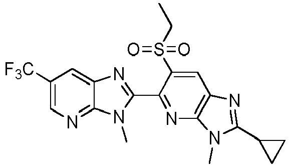

### Step A: Synthesis of 2-methylamino-5-(trifluoromethyl)pyridine

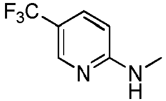

Methylamine alcohol (46.5 g, 1.5 mol) was added to a solution of 2-chloro-5-(trifluoromethyl)pyridine (90.5 g, 0.5 mol) in acetonitrile (50 mL). Stirred the mixture in a 350 mL sealed tube at 100°C overnight. The progress of the reaction was monitored by TLC and LC-MS analysis. Then concentrated the reaction mixture under vacuum to obtain white crystals, which were 2-methylamino-5-(trifluoromethyl)pyridine, yield 95%.

### Step B: synthesis of 2-methylamino-3-nitro-5-(trifluoromethyl)pyridine

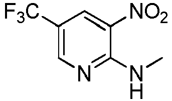

Nitric acid(18.9 g, 0.3 mol) was added dropwise to a solution of 2-methylamino-5-(trifluoromethyl)pyridine (52.8 g, 0.3 mol) in concentrated sulfuric acid (100 mL) within 30 minutes under ice bath. Then removed the ice bath and heated the reaction solution to 80°C for 3 hours. The progress of the reaction was monitored by TLC and LC-MS analysis. Then the resulting mixture was poured into ice water slowly to precipitate solid. filtered, washed with water, and dried the filter cake to give the title compound as a yellow solid (47.7 g), yield 72%.

### Step C: synthesis of 2-methylamino-3-amino-5-(trifluoromethyl)pyridine

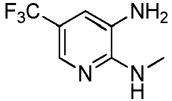

2-methylamino-3-nitro-5-(trifluoromethyl)pyridine (11.05 g, 50 mmol), palladium-carbon(5%), and tetrahydrofuran (100 mL) was stirred under hydrogen atmosphere at room temperature for 24 hours. The progress of the reaction was monitored by TLC and GC-MS analysis. The reaction mixture was filtered through Celite, and then the filtrate was concentrated under reduced pressure to obtain the title compound as reddish-brown crystals(10.9 g), with a yield of 99%.

### Step D: Synthesis of 3,6-dichloropyridine-2-carbonyl chloride

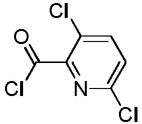

Oxalyl chloride (3.15 mL, 34.6 mmol) was added dropwise to a solution of 3,6-dichloropyridine-2-carboxylic acid (5.00 g, 24.7 mmol) and dimethylformamide (0.124 mL, 1.6 mmol) in dichloromethane (200 mL). The reaction mixture was stirred at room temperature. After 2.5 hours, added another 1 mL of oxalyl chloride to the mixture and continued stirring for 1 hour. After this time, concentrated the reaction mixture in vacuo without further purification.

### Step E: Synthesis of 3,6-dichloro-N-(2-(methylamino)-5-(trifluoromethyl)pyridin-3-yl)pyridinecarboxamide

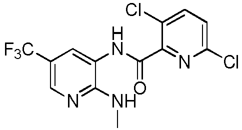

To a solution of 2-methylamino-3-amino-3-trifluoromethylpyridine (16.70 g, 87.37 mmol) in tetrahydrofuran (167.0 mL) was added triethylamine (22.32 g, 218.4 mmol). The reaction mixture was cooled to 0°C, and 3,6-dichloropyridine-2-carbonyl chloride (18.37 g, 87.37 mmol) dissolved in dichloromethane (170 mL) was added dropwise to the mixture over 1 hour at 0-10°C. After 1.5 hours, LC-MS detected the desired product. The ice bath was removed, and the reaction mixture was allowed to warm to room temperature and stirred for 12 hours. The reaction mixture was then diluted with saturated NH₄Cl(aq.), the organic phase was separated, and the aqueous phase was back-extracted with dichloromethane. The combined organic phases were washed with water, brine, dried over Na₂SO₄, filtered, and concentrated in vacuo to give a crude product. The crude product was purified by column chromatography.

### Step F: Synthesis of 2-(3,6-dichloropyridin-2-yl)-3-methyl-6-(trifluoromethyl)-3H-imidazo[4,5-b]pyridine

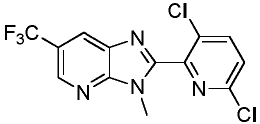

A yellow solution of 3,6-dichloro-N-(2-(methylamino)-5-(trifluoromethyl)pyridin-3-yl)pyridineamide (15.6 g, 42.75 mmol) in acetic acid (50 mL) was stirred at a bath temperature of 110°C overnight. LC-MS analysis subsequently indicated that the reaction had completed. The reaction mixture was then poured into ice water, extracted with ethyl acetate (20 mL*3), and the organic phases were combined. These organic phases were further washed with saturated sodium bicarbonate solution (20 mL*3), dried over Na₂SO₄, and purified via column chromatography to obtain 8 g of white solid. The crude yield was 54%.

### Step G: Synthesis of 2-(6-chloro-3-(ethylthio)pyridin-2-yl)-3-methyl-6-(trifluoromethyl)-3H-imidazo[4,5-b]pyridine

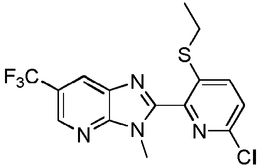

To a stirred solution of 2-(3,6-dichloropyridin-2-yl)-3-methyl-6-(trifluoromethyl)-3H-imidazo[4,5-b]pyridine (1.14 g, 3.28 mmol) in tetrahydrofuran at room temperature was added NaH (3.28 mmol, 60%). Stirred for 30 minutes, and then added ethanethiol (0.2 g, 3.28 mmol) dropwise. Stirred at room temperature until the reaction was completed. Treated the reaction mixture with saturated NH₄Cl(aq.), then extracted with ethyl acetate. Separated the organic layer, washed with water and brine, dried over Na₂SO₄, and purified the crude product by silica gel flash chromatography to obtain the title compound as a beige solid. Yield: 80%.

### Step H: Synthesis of 2-(6-chloro-3-(ethylsulfonyl)pyridin-2-yl)-3-methyl-6-(trifluoromethyl)-3H-imidazo[4,5-b]pyridine

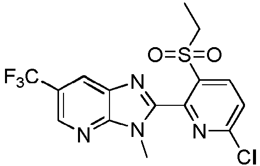

Formic acid (5.29 g, 115 mmol) and hydrogen peroxide (3.0 g, 161 mmol) were added to the product obtained in the previous step (8.556 g, 23 mmol) at room temperature. Then, stirred the mixture at room temperature for another 5 hours. After the reaction was completed, diluted it with water, added sodium bisulfite solution and stirred for one hour, followed by the addition of saturated sodium bicarbonate solution. Separated the organic phase, extracted the aqueous phase twice with dichloromethane, combined the organic phases, dried over Na₂SO₄ and purified by column chromatography to obtain 6.6 g of white solid with a yield of 74.5%.

### Step I: 5-(Ethanesulfonyl)-6-(3-methyl-6-(trifluoromethyl)-3H-imidazo[4,5-b]pyridin-2-yl)pyridin-2-amine

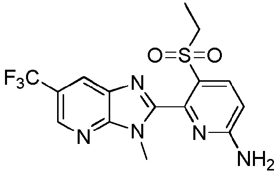

To a solution of 2-(6-chloro-3-(ethylsulfonyl)pyridin-2-yl)-3-methyl-6-(trifluoromethyl)-3*H*-imidazo[4,5-b]pyridine (0.4 g, 1 mmol) in ethanol was added 3 equivalents of aqueous ammonia. Stirred the mixture in sealed tube at 120°C overnight. LC-MS showed that the reaction was completed. Cooled the solution to room temperature, and a solid precipitated out. Filtered and washed with a small amount of methanol. Dried to obtain a yellow solid, 90% yield.

### Step J: 5-(Ethanesulfonyl)-6-(3-methyl-6-(trifluoromethyl)-3H-imidazo[4,5-b]pyridin-2-yl)pyridine-2-carboxaldehyde

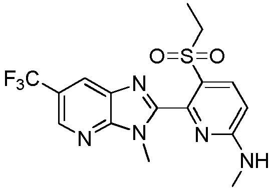

To the compound obtained in step H in methanol was added 3 equivalents of methylamine solution, Stirred the mixture in sealed tube at 120°C overnight. The progress of the reaction was monitored by LC-MS analysis, after completed, cooled the reaction solution to room temperature, a solid precipitated out. Filtered and dried it to obtain the product.

¹H NMR (400 MHz, Chloroform-d) δ 8.73 (d, *J =* 1.9 Hz, 1H), 8.28 (d, *J =* 2.0 Hz, 1H), 8.10 (d, *J =* 9.0 Hz, 1H), 6.60 (d, *J =* 8.9 Hz, 1H), 5.30 (s, 1H), 3.86 (s, 3H), 3.60 (q, *J =* 7.4 Hz, 2H), 3.03 (d, *J =* 5.1 Hz, 3H), 1.31 (t, *J =* 7.4 Hz, 3H).

### Step K: Synthesis of 5-(ethylsulfonyl)-N-methyl-6-(3-methyl-6-(trifluoromethyl)-3H-imidazo[4,5-b]pyridin-2-yl)-3-nitro-2-amino

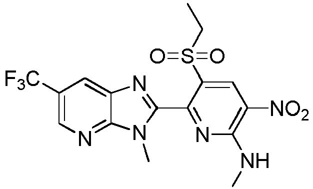

An equivalent amount of nitric acid was added dropwise to the compound obtained in step J in concentrated sulfuric acid under ice bath.. After the addition was completed, stirred at room temperature for several hours. After completed, the reaction mixture was poured into ice water to precipitate a yellow solid. Filtered and dried to obtain the product without further purification.

### Step L: Synthesis of 5-(ethylsulfonyl)-N-methyl-6-(3-methyl-6-(trifluoromethyl)-3H-imidazo[4,5-b]pyridin-2-yl)pyridine-2,3-diamine

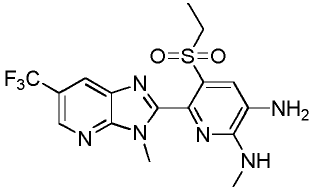

The crude product obtained in step K, palladium-carbon(5%), and tetrahydrofuran (50 mL) was stirred under hydrogen atmosphere at room temperature for overnight. The progress of the reaction was monitored by TLC and GC-MS analysis. The reaction mixture was filtered through Celite, and then the filtrate was concentrated under reduced pressure, the resulting crude product could be purified by column chromatography.

### Step M: Synthesis of target compound I-1

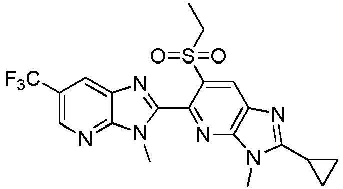

To a solution of the compound obtained in step L (1 mmol) and triethylamine (1.5 mmol) in tetrahydrofuran were added cyclopropanecarbonyl chloride (1 mmol). The mixture was stirred at room temperature. After completion, the solvent was removed, and the residue was dissolved in acetic acid. The mixture heated at 120°C and refluxed for several hours. After completion, the mixture was diluted with water and extracted with ethyl acetate, and the organic phase was dried over Na₂SO₄ and purified by column chromatography to obtain 109 mg of the title compound, with a two-step yield of 20%, m.p. 201-204°C.

¹H NMR (400 MHz, CDCl3) δ 8.79 - 8.74 (m, 1H), 8.65 (s, 1H), 8.31 (d, J = 2.0 Hz, 1H), 3.99 (s, 3H), 3.83 (s, 3H), 3.68 (q, J = 7.4 Hz, 2H), 2.14 (tt, J = 8.1, 4.8 Hz, 1H), 1.42 (dt, J = 4.8, 3.2 Hz, 2H), 1.35 - 1.28 (m, 5H).EI-MS: 465[M+H]⁺

### Example 2:

According to the method described in step M of Example 1, replacing cyclopropanecarbonyl chloride with acetyl chloride, the compound I-2 of the present invention represented by the following formula can be obtained.

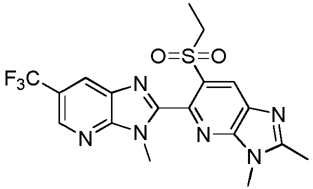

### Compound I-2 of the present invention

¹H NMR (400 MHz, CDCl₃) δ 8.79 - 8.73 (m, 2H), 8.33 (d, *J* = 2.0 Hz, 1H), 3.89 (s, 3H), 3.82 (s, 3H), 3.68 (q, *J =* 7.5 Hz, 2H), 2.79 (s, 3H), 1.33 (t, *J =* 7.4 Hz, 3H).

### Example 3:

### Synthesis of target compound I-73

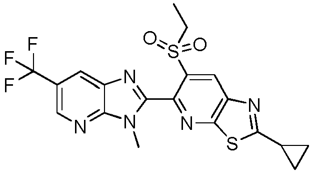

### Step A: Synthesis of intermediate compound 1

According to the method described in step E of Preparation Example 1, replacing 3,6-dichloropyridine-2-carbonyl chloride with 3-chloro-5-bromopyridine-2-carbonyl chloride, the intermediate compound 1 represented by the following formula can be obtained.

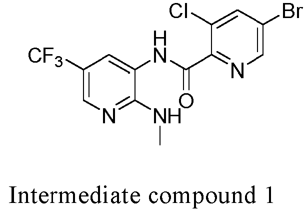

¹H NMR (400 MHz, CDCl₃) δ 9.33 (s, 1H), 8.60 (d, *J =* 1.9 Hz, 1H), 8.36 (dd, *J =* 2.2, 1.1 Hz, 1H), 8.10 (d, *J =* 1.9 Hz, 1H), 7.86 (d, *J =* 2.1 Hz, 1H), 5.03 (d, *J =* 5.9 Hz, 1H), 3.08 (d, *J =* 4.8 Hz, 3H).

### Step B: Synthesis of Intermediate Compound 2

According to the method described in step F of Preparation Example 1, intermediate compound 2 represented by the following formula can be obtained.

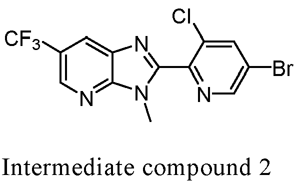

¹H NMR (400 MHz, CDCl₃) δ 8.77 (d, *J =* 1.9 Hz, 2H), 8.40 (d, *J =* 2.0 Hz, 1H), 8.16 (d, *J =* 1.9 Hz, 1H), 3.98 (d, *J =* 1.2 Hz, 3H).

### Step C: Synthesis of Intermediate Compound 3

According to the method described in step G of Preparation Example 1, intermediate Compound 3 represented by the following formula can be obtained.

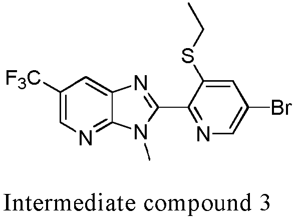

### Step D: Synthesis of Intermediate Compound 4

According to the method described in step H of Preparation Example 1, intermediate compound 4 represented by the following formula can be obtained.

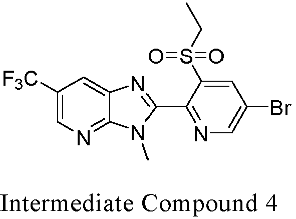

### Step E: Synthesis of Intermediate Compound 5

Intermediate Compound 4 (5 mmol) was dissolved in dried toluene (35 mL) and cesium carbonate (7.5 mmol) was then added. The mixture was degassed with argon and then tris(dibenzylideneacetone)dipalladium (0) (0.11 mmol), 2,2'-bis(diphenylphosphino)-1,1'-binaphtyle (0.2 mmol) and 2,4-Dimethoxybenzylamine (5 mmol) were added. The mixture was stirred under argon at 105°C (oil bath). The dark violet color of the mixture changed to pale yellow within 15 min. After 20 h, the reaction was cooled, and the solvent was removed under reduced pressure. The residue was purified on 50 g of silica gel, eluent 50% ethyl acetate in hexane. The intermediate compound 5 represented by the following formula can be obtained.

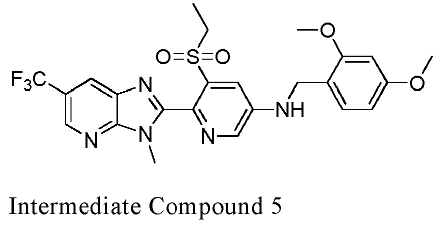

### Step F: Synthesis of intermediate compound 6

Intermediate compound 5 (3.31 mmol) was dissolved in dichloromethane (20 mL) at room temperature and trifluoroacetic acid (3.31 mmol) was then added. The orange-red solution was stirred at room temperature for 5 h. The volatiles were removed under reduced pressure. The residue was alkalized with saturated solution of NaHCO₃ and extracted with ethyl acetate. The organic phase was separated, dried and concentrated. The residue was purified on 40 g of silica gel, eluent ethyl acetate to give intermediate compound 6 represented by the following formula.

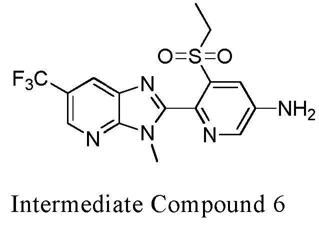

### Step G: Synthesis of intermediate compound 7

Intermediate compound 6 (1 mmol) was dissolved in acetic acid glacial (10 mL) and then anhydrous sodium acetate (1 mmol) was added. Then bromine (1.2 mmol) dissolved in acetic acid (5 mL) was added dropwise at room temperature. The mixture was stirred at room temperature. After completion, The reaction mixture was washed with sodium thiosulfate solution and extracted with ethyl acetate. Finally, the organic phase was dried over Na₂SO₄. The residue was purified on silica gel, eluent 50% ethyl acetate in hexane to give intermediate compound 7 represented by the following formula.

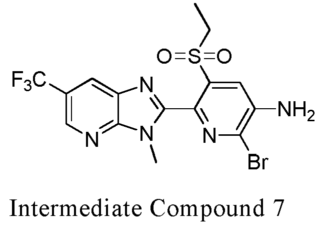

### Step H: Synthesis of Intermediate Compound 8

Intermediate compound 7 (1 mmol) was dissolved in tetrahydrofuran, then cyclopropanecarbonyl chloride (1 mmol) and triethylamine (1.5 mmol) were added. The mixture was stirred at room temperature. After the reaction was completed, purified it by column chromatography to obtain intermediate compound 8, which was a white solid represented by the following formula.

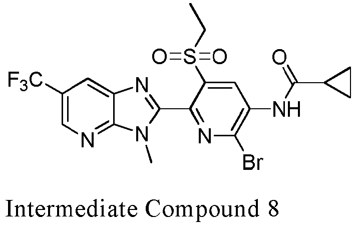

¹H NMR (400 MHz, Chloroform-d) δ 9.44 (d, *J =* 1.6 Hz, 1H), 8.75 (d, *J =* 2.0 Hz, 1H), 8.30 (d, *J =* 2.0 Hz, 1H), 8.12 (s, 1H), 3.91 (d, *J* = 1.6 Hz, 3H), 3.81 (q, *J =* 7.5 Hz, 2H), 1.74 - 1.68 (m, 1H), 1.39 (t, *J =* 7.5 Hz, 3H), 1.26 - 1.22 (m, 3H), 1.06 (dt, *J =* 7.5, 3.5 Hz, 2H).

### Step I: Synthesis of intermediate compound 9

Intermediate compound 8 (1 mmol) was dissolved in tetrahydrofuran (15 mL), followed by the addition of Lawesson's reagent (1.5 mmol) and stirring under reflux. After the reaction was completed, the solvent was evaporated, and the residue was purified by column chromatography to give intermediate compound 9, represented by the following formula, as a light yellow solid with a yield of 40%.

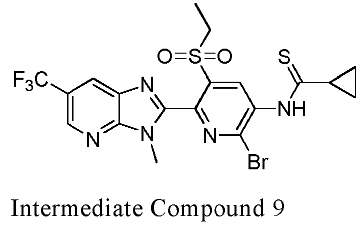

### Step J: Synthesis of target compound I-73

Intermediate compound 9 (1 mmol) was dissolved in 1,4-dioxane (15 mL), then cesium carbonate (1.5 mmol) was added. The reaction mixture was stirred and heated at 80°Cin oil bath. After the reaction was completed, the mixture was diluted with water and extracted with ethyl acetate.. The organic phase was dried over Na₂SO₄. Finally, purified by column chromatography to obtain the compound I-73 of the present invention represented by the following formula as a white solid with a yield of 80%, m.p. 216-219°C.

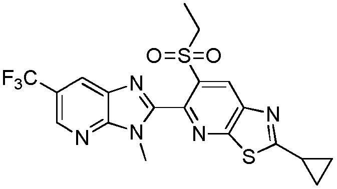

### Compound I-73 of the present invention

¹H NMR (400 MHz, CDCl₃) δ 8.84 (s, 1H), 8.77 (d, *J =* 1.9 Hz, 1H), 8.34 (d, *J* = 2.1 Hz, 1H), 3.86 (s, 3H), 3.75 (q, *J =* 7.4 Hz, 2H), 2.51 (tt, *J =* 8.1, 5.3 Hz, 1H), 1.46 - 1.41 (m, 4H), 1.35 (t, *J =* 7.4 Hz, 3H).EI-MS: 468[M+H]⁺.

### Example 4:

### Synthesis of target compound I-37

Intermediate compound 8 (1 mmol) was dissolved in 1,4-dioxane (15 mL), then CuI was added (1.5 mmol) and stirred under reflux in a nitrogen atmosphere. After the reaction was completed, distilled off the solvent, and the residue was purified by column chromatography to give the compound I-37 of the present invention, represented by the following formula, as a white solid, with a yield of 40%. m.p. 224-227°C.

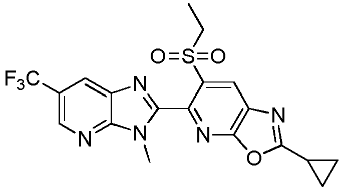

### Compound I-37 of the present invention

¹H NMR (400 MHz, CDCl₃) δ 8.77 (dd, *J =* 2.0, 0.8 Hz, 1H), 8.69 (s, 1H), 8.31 (d, *J =* 2.0 Hz, 1H), 3.87 (s, 3H), 3.83 (q, *J =* 7.4 Hz, 2H), 2.35 (tt, *J =* 8.2, 4.9 Hz, 1H), 1.50 - 1.45 (m, 2H), 1.42 - 1.39 (m, 2H), 1.36 (d, *J =* 7.5 Hz, 3H).EI-MS: 452[M+H]⁺

### Example 5:

### Synthesis of target compound I-109

### Step A: Synthesis of intermediate compound 10

According to the method described in step I of Preparation Example 3, the substrate (1 mmol) was dissolved in tetrahydrofuran (15 mL), followed by the addition of Lawesson's reagent (1.5 mmol) and stirring under reflux. After the reaction was completed, the solvent was evaporated, and the residue was purified by column chromatography to give the intermediate compound 10 represented by the following formula, with a yield of 70%.

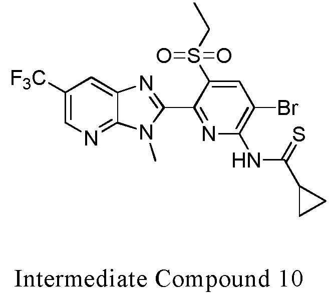

### Step B: Synthesis of target compound I-109

Intermediate compound 10 (1 mmol) was dissolved in 1,4-dioxane (15 mL), followed by the addition of cesium carbonate (1.5 mmol). The mixture was stirred and heated at 80°Cin oil bath. Upon completion of the reaction, the mixture was diluted with water and extracted with ethyl acetate. The organic phase was dried over anhydrous sodium sulfate. Finally, the residue was purified by column chromatography to give compound I-109 of the present invention, represented by the following formula, with a yield of 85%. m.p. 207-210°C.

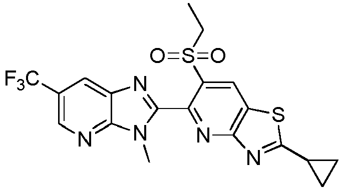

### Compound I-109 of the present invention

¹H NMR (400 MHz, CDCl₃) δ 8.99 (s, 1H), 8.79-8.73 (m, 2H), 8.30 (dd, *J* = 8.8, 1.9 Hz, 2H), 3.94 (s, 3H), 3.93-3.88 (m, 2H), 2.52 (tt, *J =* 8.0, 4.7 Hz, 1H), 1.59-1.54 (m, 2H), 1.47 (dt, *J =* 8.1, 3.4 Hz, 2H), 1.41-1.37 (m, 3H).EI-MS: 468[M+H]⁺.

Similar to the example and based on the aforementioned preparation method, the following compound of formula (I) was synthesized:

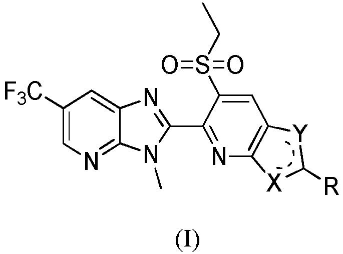

**Table 2**

| Comp. No. | X | Y | R | m.p. (°C) | EI-MS: [M+H]⁺ |
|---|---|---|---|---|---|
| I-1 | N-CH₃ | N | cyclopropyl | 201-204 | 465 |
| I-2 | N-CH₃ | N | -CH₃ | 241-244 | 463 |
| I-3 | N-CH₃ | N | -Ph | 189-191 | 501 |
| I-4 | N-CH₃ | N | -2-F-Ph | | |
| I-5 | N-CH₃ | N | -2,4-F-Ph | 206-209 | 537 |
| I-6 | N-CH₃ | N | -CH₂CH₃ | | |
| I-7 | N-CH₃ | N | isopropyl | 206-209 | 467 |
| I-8 | N-CH₃ | N | -CH₂CH₂CH₃ | 205-207 | 467 |
| I-9 | N-CH₃ | N | tert-butyl | 196-199 | 463 |
| I-10 | N-CH₃ | N | cyclobutyl | 226-228 | 463 |
| I-11 | N-CH₃ | N | cyclopentyl | | |
| I-12 | N-CH₃ | N | cyclohexyl | | |
| I-13 | N-CH₃ | N | -3-F-Ph | | |
| I-14 | N-CH₃ | N | -4-F-Ph | 182-184 | 519 |
| I-15 | N-CH₃ | N | propenyl | | |
| I-16 | N-CH₃ | N | difluoromethyl | 182-184 | 475 |
| I-17 | N-CH₃ | N | cyclopropanecarbonitrile | 255-258 | 490 |
| I-18 | N-CH₃ | N | -2-Cl-Ph | | |
| I-19 | N-CH₃ | N | -2-Br-Ph | | |
| I-20 | N-CH₃ | N | -2-CH₃-Ph | | |
| I-21 | N-CH₃ | N | -2-CF₃-Ph | | |
| I-22 | N-CH₃ | N | -2-OCH₃-Ph | | |
| I-23 | N-CH₃ | N | -3-OCF₃-Ph | | |
| I-24 | N-CH₃ | N | -3,4-OCH₃-Ph | | |
| I-25 | N-CH₃ | N | ethynyl | | |
| I-26 | N-CH₃ | N | isobutyl | | |
| I-27 | N-CH₃ | N | -CH₂Cl | | |
| I-28 | N-CH₃ | N | trifluoromethyl | | |
| I-29 | N-CH₃ | N | ethenyl | 235-237 | 451 |
| I-30 | N-CH₃ | N | propynyl | | |
| I-31 | N-CH₃ | N | butenyl | | |
| I-32 | N-CH₃ | N | butynyl | | |
| I-33 | N-CH₃ | N | -2-thiophenyl | | |
| I-34 | N-CH₃ | N | -3-Py | | |
| I-35 | N-CH₃ | N | -2-Cl-Py | | |
| I-36 | N-CH₃ | N | -5-Br-Py | | |
| I-37 | O | N | cyclopropyl | 224-227 | 452 |
| I-38 | O | N | -CH₃ | | |
| I-39 | O | N | -Ph | | |
| I-40 | O | N | -2-F-Ph | | |
| I-41 | O | N | -2,4-F-Ph | | |
| I-42 | O | N | -CH₂CH₃ | | |
| I-43 | O | N | isopropyl | | |
| I-44 | O | N | -CH₂CH₂CH₃ | | |
| I-45 | O | N | tert-butyl | | |
| I-46 | O | N | cyclobutyl | | |
| I-47 | O | N | Cyclopentyl | | |
| I-48 | O | N | cyclohexyl | | |
| I-49 | O | N | -3-F-Ph | | |
| I-50 | O | N | -4-F-Ph | | |
| I-51 | O | N | propenyl | | |
| I-52 | O | N | difluoromethyl | | |
| I-53 | O | N | cyclopropanecarbonitrile | | |
| I-54 | O | N | -2-Cl-Ph | | |
| I-55 | O | N | -2-Br-Ph | | |
| I-56 | O | N | -2-CH₃-Ph | | |
| I-57 | O | N | -2-CF₃-Ph | | |
| I-58 | O | N | -2-OCH₃-Ph | | |
| I-59 | O | N | -3-OCF₃-Ph | | |
| I-60 | O | N | -3,4-OCH₃-Ph | | |
| I-61 | O | N | ethynyl | | |
| I-62 | O | N | isobutyl | | |
| I-63 | O | N | -CH₂Cl | | |
| I-64 | O | N | trifluoromethyl | | |
| I-65 | O | N | ethenyl | | |
| I-66 | O | N | propynyl | | |
| I-67 | O | N | butenyl | | |
| I-68 | O | N | butynyl | | |
| I-69 | O | N | -2-thiophenyl | | |
| I-70 | O | N | -3-Py | | |
| I-71 | O | N | -2-Cl-Py | | |
| I-72 | O | N | -5-Br-Py | | |
| I-73 | S | N | cyclopropyl | 216-219 | 468 |
| I-74 | S | N | -CH₃ | 150-152 | 442 |
| I-75 | S | N | -Ph | | |
| I-76 | S | N | -2-F-Ph | | |
| I-77 | S | N | -2,4-F-Ph | | |
| I-78 | S | N | -CH₂CH₃ | | |
| I-79 | S | N | isopropyl | | |
| I-80 | S | N | -CH₂CH₂CH₃ | | |
| I-81 | S | N | tert-butyl | | |
| I-82 | S | N | cyclobutyl | | |
| I-83 | S | N | Cyclopentyl | | |
| I-84 | S | N | cyclohexyl | | |
| I-85 | S | N | -3-F-Ph | | |
| I-86 | S | N | -4-F-Ph | 212-213 | 522 |
| I-87 | S | N | propenyl | | |
| I-88 | S | N | difluoromethyl | 204-205 | 478 |
| I-89 | S | N | cyclopropanecarbonitrile | | |
| I-90 | S | N | -2-Cl-Ph | | |
| I-91 | S | N | -2-Br-Ph | | |
| I-92 | S | N | -2-CH₃-Ph | | |
| I-93 | S | N | -2-CF₃-Ph | | |
| I-94 | S | N | -2-OCH₃-Ph | | |
| I-95 | S | N | -3-OCF₃-Ph | | |
| I-96 | S | N | -3,4-OCH₃-Ph | | |
| I-97 | S | N | ethynyl | | |
| I-98 | S | N | isobutyl | | |
| I-99 | S | N | -CH₂Cl | | |
| I-100 | S | N | trifluoromethyl | 180-182 | 496 |
| I-101 | S | N | ethenyl | | |
| I-102 | S | N | propynyl | | |
| I-103 | S | N | butenyl | | |
| I-104 | S | N | butynyl | | |
| I-105 | S | N | -2-thiophenyl | | |
| I-106 | S | N | -3-Py | | |
| I-107 | S | N | -2-Cl-Py | | |
| I-108 | S | N | -5-Br-Py | | |
| I-109 | N | S | cyclopropyl | 207-210 | 468 |
| I-110 | N | S | -CH₃ | | |
| I-111 | N | S | -Ph | | |
| I-112 | N | S | -2-F-Ph | | |
| I-113 | N | S | -2,4-F-Ph | | |
| I-114 | N | S | -CH₂CH₃ | | |
| I-115 | N | S | isopropyl | | |
| I-116 | N | S | -CH₂CH₂CH₃ | | |
| I-117 | N | S | tert-butyl | | |
| I-118 | N | S | cyclobutyl | | |
| I-119 | N | S | Cyclopentyl | | |
| I-120 | N | S | cyclohexyl | | |
| I-121 | N | S | -3-F-Ph | | |
| I-122 | N | S | -4-F-Ph | | |
| I-123 | N | S | propenyl | | |
| I-124 | N | S | difluoromethyl | | |
| I-125 | N | S | cyclopropanecarbonitrile | | |
| I-126 | N | S | -2-Cl-Ph | | |
| I-127 | N | S | -2-Br-Ph | | |
| I-128 | N | S | -4-CH₃-Ph | | |
| I-129 | N | S | -2-CF₃-Ph | | |
| I-130 | N | S | -2-OCH₃-Ph | | |
| I-131 | N | S | -3-OCF₃-Ph | | |
| I-132 | N | S | -3,4-OCH₃-Ph | | |
| I-133 | N | S | ethynyl | | |
| I-134 | N | S | isobutyl | | |
| I-135 | N | S | -CH₂Cl | | |
| I-136 | N | S | trifluoromethyl | | |
| I-137 | N | S | ethenyl | | |
| I-138 | N | S | propynyl | | |
| I-139 | N | S | butenyl | | |
| I-140 | N | S | butynyl | | |
| I-141 | N | S | -2-thiophenyl | | |
| I-142 | N | S | -3-Py | | |
| I-143 | N | S | -2-Cl-Py | | |
| I-144 | N | S | -5-Br-Py | | |

The nuclear magnetic resonance data of the preferred compound mentioned above are shown in Table 3:

**Table 3**

| Comp. No. | ¹H NMR |
|---|---|
| I-1 | ¹H NMR (400 MHz, CDCl₃) δ 8.79 - 8.74 (m, 1H), 8.65 (s, 1H), 8.31 (d, *J* = 2.0 Hz, 1H), 3.99 (s, 3H), 3.83 (s, 3H), 3.68 (q, *J =* 7.4 Hz, 2H), 2.14 (tt, *J =* 8.1, 4.8 Hz, 1H), 1.42 (dt, *J =* 4.8, 3.2 Hz, 2H), 1.35 - 1.28 (m, 5H). |
| I-2 | ¹H NMR (400 MHz, CDCl₃) δ 8.79 - 8.73 (m, 2H), 8.33 (d, *J =* 2.0 Hz, 1H), 3.89 (s, 3H), 3.82 (s, 3H), 3.68 (q, *J =* 7.5 Hz, 2H), 2.79 (s, 3H), 1.33 (t, *J =* 7.4 Hz, 3H). |
| I-3 | ¹H NMR (400 MHz, CDCl₃) δ 8.86 - 8.80 (m, 1H), 8.66 (s, 1H), 8.41 (d, *J=* 2.0 Hz, 1H), 7.91 - 7.82 (m, 2H), 7.68 - 7.59 (m, 3H), 4.30 (s, 3H), 3.97 (s, 3H), 3.44 (q, *J =* 7.4 Hz, 2H), 1.32 (t, *J =* 7.5 Hz, 3H). |
| I-5 | ¹H NMR (400 MHz, CDCl₃) δ 8.90 (s, 1H), 8.78 (s, 1H), 8.33 (s, 1H), 7.82 (q, *J =* 7.8 Hz, 1H), 7.23 - 7.02 (m, 2H), 3.99 - 3.84 (m, 6H), 3.76 (q, *J=* 7.4 Hz, 2H), 1.36 (t, *J =* 7.4 Hz, 3H). |
| I-7 | ¹H NMR (400 MHz, CDCl₃) δ 8.70 (s, 2H), 8.24 (d, *J =* 2.0 Hz, 1H), 3.84 (s, 3H), 3.77 (s, 3H), 3.62 (q, *J =* 7.4 Hz, 2H), 3.27 (d, *J =* 6.7 Hz, 1H), 1.46 (d, *J =* 6.8 Hz, 6H), 1.24 (d, *J =* 7.5 Hz, 4H). |
| I-8 | ¹H NMR (400 MHz, CDCl₃) δ 8.80 - 8.72 (m, 2H), 8.32 (dd, *J =* 2.1, 0.7 Hz, 1H), 3.89 (s, 3H), 3.84 (s, 3H), 3.70 (q, *J =* 7.4 Hz, 2H), 3.00 (dd, *J =* 8.1, 7.2 Hz, 2H), 2.06 - 1.96 (m, 2H), 1.33 (t, *J =* 7.4 Hz, 4H), 1.14 (t, *J =* 7.4 Hz, 3H). |
| I-9 | ¹H NMR (400 MHz, CDCl₃) δ 8.75 - 8.67 (m, 2H), 8.25 (d, *J =* 2.0 Hz, 1H), 4.01 (s, 3H), 3.77 (s, 3H), 1.56 (s, 9H). |
| I-10 | ¹H NMR (400 MHz, CDCl₃) δ 8.82 - 8.72 (m, 2H), 8.31 (d, *J=* 2.0 Hz, 1H), 3.92 - 3.85 (m, 1H), 3.82 (d, *J =* 10.8 Hz, 6H), 3.69 (q, *J =* 7.4 Hz, 2H), 2.74 - 2.63 (m, 2H), 2.62 - 2.52 (m, 2H), 2.26 (dt, *J =* 11.3, 8.9 Hz, 1H), 2.18 - 2.09 (m, 1H), 1.32 (t, *J =* 6.8 Hz, 3H). |
| I-14 | ¹H NMR (400 MHz, CDCl₃) δ 8.86 (s, 1H), 8.78 (dd, *J* = 1.9*,* 0.9 Hz, 1H), 8.33 (d, *J =* 1.9 Hz, 1H), 7.98 - 7.89 (m, 2H), 7.33 (t, *J =* 8.6 Hz, 2H), 4.05 (s, 3H), 3.87 (s, 3H), 3.75 (q, *J =* 7.4 Hz, 2H), 1.36 (t, *J =* 7.4 Hz, 3H). |
| I-16 | ¹H NMR (400 MHz, CDCl₃) δ 8.90 (s, 1H), 8.70 (d, *J =* 1.8 Hz, 1H), 8.26 (d, *J =* 2.0 Hz, 1H), 6.94 (t, *J =* 52.1 Hz, 1H), 4.05 (s, 3H), 3.79 (s, 3H), 3.70 (q, *J =* 7.4 Hz, 2H), 1.27 (t, *J =* 7.4 Hz, 3H). |
| I-17 | ¹H NMR (400 MHz, CDCl₃) δ 8.78 (d, *J =* 2.0 Hz, 2H), 8.32 (d, *J =* 2.0 Hz, 1H), 4.20 (s, 3H), 3.85 (s, 3H), 3.74 (q, *J =* 7.4 Hz, 2H), 2.08 - 2.03 (m, 2H), 2.02 - 1.97 (m, 2H), 1.33 (t, *J =* 7.4 Hz, 3H). |
| I-28 | ¹H NMR (400 MHz, CDCl₃) δ 9.03 (s, 1H), 8.83 - 8.76 (m, 1H), 8.34 (d, *J =* 1.9 Hz, 1H), 4.12 (d, *J =* 1.2 Hz, 3H), 3.87 (s, 3H), 3.77 (q, *J =* 7.4 Hz, 2H), 1.35 (t, *J =* 7.4 Hz, 3H). |
| I-29 | ¹H NMR (400 MHz,CDCl₃) δ 8.79 (s, 1H), 8.77 (dd, *J =* 2.0, 0.8 Hz, 1H), 8.31 (dd, *J* = 1.9, 0.7 Hz, 1H), 6.93 (dd, *J* = 17.1, 10.6 Hz, 1H), 6.82 (dd, *J =* 17.2, 1.6 Hz, 1H), 6.01 (dd, *J =* 10.6, 1.6 Hz, 1H), 3.97 (s, 3H), 3.84 (s, 3H), 3.72 (q, *J =* 7.4 Hz, 2H), 1.33 (t, *J =* 7.4 Hz, 3H). |
| I-37 | ¹H NMR (400 MHz, CDCl₃) δ 8.77 (dd, *J =* 2.0, 0.8 Hz, 1H), 8.69 (s, 1H), 8.31 (d, *J =* 2.0 Hz, 1H), 3.87 (s, 3H), 3.83 (q, *J =* 7.4 Hz, 2H), 2.35 (tt, *J =* 8.2, 4.9 Hz, 1H), 1.50 - 1.45 (m, 2H), 1.42 - 1.39 (m, 2H), 1.36 (d, *J =* 7.5 Hz, 3H). |
| I-73 | ¹H NMR (400 MHz, CDCl₃) δ 8.84 (s, 1H), 8.77 (d, *J =* 1.9 Hz, 1H), 8.34 (d, *J* = 2.1 Hz, 1H), 3.86 (s, 3H), 3.75 (q, *J =* 7.4 Hz, 2H), 2.51 (tt, *J =* 8.1, 5.3 Hz, 1H), 1.46-1.41 (m, 4H), 1.35 (t, *J =* 7.4 Hz, 3H). |
| I-74 | ¹H NMR (400 MHz, CDCl₃) δ 8.96 (s, 1H), 8.78 (dd, *J =* 2.0, 0.8 Hz, 1H), 8.34 (dd, *J =* 2.0, 0.7 Hz, 1H), 3.88 (s, 3H), 3.79 (t, *J* = 7.4 Hz, 2H), 3.00 (s, 3H), 1.37 (t, *J =* 7.4 Hz, 3H). |
| I-86 | ¹H NMR (400 MHz, CDCl₃) δ 9.04 (s, 1H), 8.79 (dd, *J =* 1.9*,* 0.8 Hz, 1H), 8.34 (dd, *J =* 2.0, 0.7 Hz, 1H), 8.23 - 8.16 (m, 2H), 7.32 - 7.27 (m, 2H), 3.92 (s, 3H), 3.88 - 3.82 (m, 2H), 1.41 (t, *J* = 7.4 Hz, 3H). |
| I-88 | ¹H NMR (400 MHz, CDCl₃) δ 9.21 (s, 1H), 8.83 - 8.78 (m, 1H), 8.35 (d, *J =* 2.0 Hz, 1H), 7.00 (t, *J =* 54.0 Hz, 1H), 3.92 (s, 3H), 3.89 (t, *J =* 7.4 Hz, 2H), 1.41 (t, *J =* 7.4 Hz, 3H). |
| I-100 | ¹H NMR (400 MHz, CDCl₃) δ 9.29 (s, 1H), 8.81 (dd, *J =* 2.0, 0.8 Hz, 1H), 8.35 (dd, *J =* 2.0, 0.8 Hz, 1H), 3.95 - 3.87 (m, 6H), 1.41 (t, *J =* 7.4 Hz, 3H). |
| I-109 | ¹H NMR (400 MHz, CDCl₃) δ 8.99 (s, 1H), 8.79-8.73 (m, 2H), 8.30 (dd, *J =* 8.8, 1.9 Hz, 2H), 3.94 (s, 3H), 3.93-3.88 (m, 2H), 2.52 (tt, *J =* 8.0, 4.7 Hz, 1H), 1.59-1.54 (m, 2H), 1.47 (dt, *J =* 8.1, 3.4 Hz, 2H), 1.41-1.37 (m, 3H). |

### Biological Examples:

This test case is used to illustrate the insecticidal activity inhibition (%) of compounds with the structure shown in formula (I) (dosage of 100ppm).

### Example 1: Activity against Myzus persicae (Green peach aphid):

Roots of pea seedlings infested with an aphid population of mixed ages were placed directly into agueous test solutions prepared from 10'000 DMSO stock solutions. The samples were assessed for mortality 6 days after placing seedlings into test solutions.

The following compounds resulted in at least 90% mortality at a test rate of 100 ppm:
I-1, I-2, I-3, I-5, I-7, I-8, I-9, I-10, I-14, I-16, I-17, I-29, I-37, I-73 and I-109.

### Example 2: Activity against Plutella xylostella:

24-well microtiter plates with artificial diet were treated with agueous test solutions prepared from 10'000 ppm DMSO stock solutions by pipetting. After drying, the plates were infested with L2 larvae (10 to 15 per well). The samples were assessed for mortality and growth inhibition in comparison to untreated samples 5 days after infestation.

The following compounds gave an effect of at least 90% in at least one of the two categories (mortality or growth inhibition) at an application rate of 100 ppm:
I-1, I-2, I-3, I-5, I-7, I-8, I-9, I-10, I-14, I-16, I-17, I-29, I-37, I-73 and I-109.

Further, The following compounds gave an effect of 100% at an application rate of 4 ppm:
I-73 and I-109

### Example 3: Activity against Leucania separate:

Maize sprouts placed onto an agar layer in 24-well microtiter plates were treated with agueous test solutions prepared from 10'000 ppm DMSO stock solutions by spraying. After drying, the plates were infested with L2 larvae (6 to 10 per well). The samples were assessed for mortality and growth inhibition in comparison to untreated samples 4 days after infestation.

The following compounds gave an effect of at least 90% in at least one of the two categories (mortality or growth inhibition) at an application rate of 100 ppm:
I-1, I-2, I-3, I-5, I-7, I-8, I-9, I-10, I-14, I-16, I-17, I-29, I-37, I-73 and 1-109.

### Example 4: Activity against Nilaparvata lugens:

Cotton leaf discs were placed on agar in 24-well microtiter plates and sprayed with agueous test solutions prepared from 10'000 ppm DMSO stock solutions. After drying the leaf discs were infested with adult *Nilaparvata lugens.* The samples were checked for mortality 6 days after incubation.

The following compounds resulted in at least 90% mortality at a test rate of 100 ppm:
I-1, I-2, I-3, I-5, I-7, I-8, I-9, I-10, I-14, I-16, I-17, I-29, I-37, I-73, I-100 and I-109.

Further, The following compounds gave an effect of 95% at an application rate of 45 ppm:
I-73, I-100 and I-109.

### Example 5: Activity against Laphygma exigua:

24-well microtiter plates with artificial diet were treated with agueous test solutions prepared from 10'000 ppm DMSO stock solutions by pipetting. After drying, the plates were infested with L2 larvae (10 to 15 per well). The samples were assessed for mortality and growth inhibition in comparison to untreated samples 5 days after infestation.

The following compounds gave an effect of at least 90% in at least one of the two categories (mortality or growth inhibition) at an application rate of 100 ppm:
I-1, I-2, I-3, I-5, I-7, I-8, I-9, I-10, I-14, I-16, I-17, I-29, I-37, I-73, I-74, I-86 and I-109.

Further, The following compounds gave an effect of 100% at an application rate of 4 ppm:
I-73 and I-109.

### Example 6: Activity against Aphis craccivora Koch:

Roots of pea seedlings infested with an aphid population of mixed ages were placed directly into agueous test solutions prepared from 10'000 DMSO stock solutions. The samples were assessed for mortality 6 days after placing seedlings into test solutions.

The following compounds resulted in at least 90% mortality at a test rate of 100 ppm:
I-1, I-2, I-3, I-5, I-7, I-8, I-9, I-10, I-14, I-16, I-17, I-29, I-37, I-73, I-74, I-86, I-88 and I-109.

Further, The following compounds gave an effect of 100% at an application rate of 4 ppm:
I-1, I-37, I-73, I-100 and I-109.

### Example 7: Activity against Chilo suppressalis:

Take 5~6 rice seedlings, each 3~4 cm in height, soak them thoroughly in the prepared solution, After drying, the plates were infested with L2 larvae (10 per well). Keep them in an observation room for rearing and observation. Use 0.1% warm water as a blank control. Incubate them in an observation room at 25 to 27 °C. After 96 hours, assess the samples based on mortality rates

The following compounds gave an effect of 100% at an application rate of 5 ppm:
I-73 and I-109.

What has been described above are merely preferred embodiments of the present invention. It should be noted that for those skilled in the art, several variations and improvements can be made without departing from the inventive concept of the present invention, and all of these fall within the scope of protection of the present invention.

## Claims

1. A fused ring compound with a sulfur-containing substituent of formula (I): 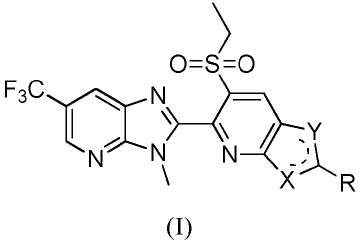 wherein,
X is selected from N, NR₁, O or S;
Y is selected from N or S; when Y is selected from N, X is selected from NR₁, O or S; when Y is selected from S, X is selected from N;
R₁ is selected from H, C_{1~6} alkyl, C_{1~6} halogenated alkyl, C_{3~6} cycloalkyl, C_{3~6} halogenated cycloalkyl;
R is selected from H, C_{1~6} alkyl, C_{1~6} substituted alkyl, C_{3~6} cycloalkyl, C_{3~6} substituted cycloalkyl, C_{2~6} epoxy, C₂₋₆ alkenyl, C₂₋₆ alkynyl, phenyl, substituted phenyl, C_{5~6} heterocycle or substituted heterocycle;
the substituent of substituted alkyl or substituted cycloalkyl is selected from halogen, C_{1~2} alkyl, C_{1~2} alkoxy, C_{1~2} halogenated alkoxy, C_{1~2} halogenated alkyl, cyano or ester group;
the substituted phenyl refers to phenyl substituted with 1, 2 or 3 substituents, the substituent is selected from halogen, C_{1~2} alkoxy, C_{1~2} halogenated alkoxy, cyano, C_{1~6} alkyl or C_{1~6} halogenated alkyl;
the heterocycle is selected from five-membered or six-membered monocyclic ring containing at least one non-carbon ring atom, including aromatic ring and non-aromatic hydrocarbon;
the substituted heterocycle refers to heterocycle substituted with 1, 2 or 3 substituents, the substituent of the substituted heterocycle is selected from halogen, C_{1~6} alkyl, C_{1~6} halogenated alkyl, C_{1~6} alkoxy or C_{1~6} alkylamide group.

2. The fused ring compound with a sulfur-containing substituent according to claim 1, wherein,
X is selected from N, NR₁, O or S;
Y is selected from N or S; when Y is selected from N, X is selected from NR₁, O or S; when Y is selected from S, X is selected from N;
R₁ is selected from H, C_{1~6} alkyl, trifluoromethyl or cyclopropyl;
R is selected from C_{1~6} alkyl, C_{1~6} halogenated alkyl, C_{3~6} cycloalkyl, C₂₋₆ alkenyl, C₂₋₆ alkynyl, phenyl, substituted phenyl, C_{5~6} heterocycle or substituted heterocycle;
the substituted phenyl refers to phenyl substituted with 1, 2 or 3 substituents, the substituent is selected from halogen, methoxyl, cyano, ethoxyl, methyl, ethyl or trifluoromethyl;
the heterocycle is selected from piperazinyl, pyrrolidinyl, dioxanyl, morpholinyl, tetrahydrofuranyl, pyridyl, furanyl, imidazolyl, pyrimidinyl, oxazolyl, isoxazolyl, tetrazolyl, triazolyl, thiadiazolyl or thienyl;
the substituted heterocycle refers to heterocycle substituted with 1, 2 or 3 substituents, the substituent of the substituted heterocycle is selected from halogen, methyl, ethyl, methoxyl, trifluoromethyl or difluoromethyl.

3. The fused ring compound with a sulfur-containing substituent according to claim 1, wherein,
R is selected from C_{1~6} alkyl, C_{1~6} halogenated alkyl, C_{3~6} cycloalkyl, C₂₋₆ alkenyl, C₂₋₆ alkynyl, phenyl, substituted phenyl, C_{5~6} heterocycle or substituted heterocycle;
the substituted phenyl refers to phenyl substituted with 1, 2 or 3 substituents, the substituent of the substituted phenyl is selected from halogen, methoxyl, cyano, methyl or trifluoromethyl;
the heterocycle is selected from 2-thienyl, pyridyl, 2-chloropyridyl or 5-bromopyridyl;
the substituted heterocycle refers to heterocycle substituted with 1, 2 or 3 substituents, the substituent of the substituted heterocycle is selected from halogen, methyl, methoxyl or trifluoromethyl.

4. The fused ring compound with a sulfur-containing substituent according to claim 1, wherein,
R₁ is selected from methyl or ethyl;
R is selected from C_{1~6} alkyl, C_{1~3} halogenated alkyl, C_{3~6} cycloalkyl, C₂₋₆ alkenyl, C₂₋₆ alkynyl, phenyl, substituted phenyl, C_{5~6} heterocycle or substituted heterocycle;
the substituted phenyl refers to phenyl substituted with 1, 2 or 3 substituents, the substituent is selected from halogen, methoxyl, cyano, methyl or trifluoromethyl;
the heterocycle is selected from 2-thienyl, pyridyl, 2-chloropyridyl or 5-bromopyridyl;
the substituted heterocycle refers to heterocycle substituted with 1, 2 or 3 substituents, the substituent of the substituted heterocycle is selected from halogen, methyl, methoxyl or trifluoromethyl.

5. The fused ring compound with a sulfur-containing substituent according to claim 1, wherein,
R is selected from methyl, ethyl, trifluoromethyl, difluoromethyl, chloromethyl, cyclopropyl, heptafluoroisopropyl, isopropyl, n-propyl, isobutyl, tert-butyl, cyclobutyl, cyclopentyl, cyclohexyl, vinyl, ethynyl, propenyl, butenyl, propynyl, butynyl, phenyl, cyanocyclopropyl, 4-fluorophenyl, 2-fluorophenyl, 3-fluorophenyl, 2,4-difluorophenyl, 2-thienyl, 2-pyridyl, 2-chloropyridyl or 5-bromopyridyl.

6. The fused ring compound with a sulfur-containing substituent according to claim 1, comprising compounds of formula (I-a), (I-b), (I-c) and (I-d): 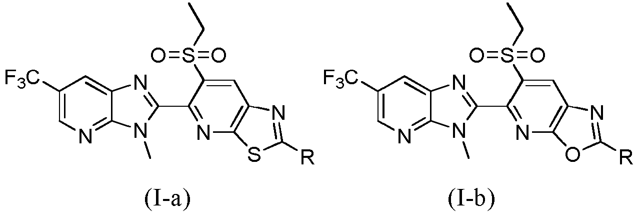 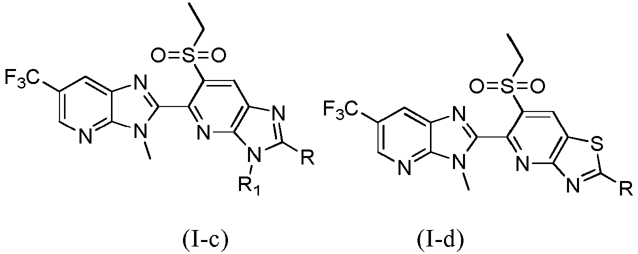 wherein,
R₁ is selected from methyl or ethyl;
R is selected from methyl, trifluoromethyl, difluoromethyl, chloromethyl, cyclopropyl, heptafluoroisopropyl, isopropyl, n-propyl, isobutyl, tert-butyl, cyclobutyl, cyclopentyl, cyclohexyl, vinyl, ethynyl, propenyl, butenyl, propargyl, butynyl, phenyl, cyanocyclopropyl, 4-fluorophenyl, 2-fluorophenyl, 3-fluorophenyl, 2,4-difluorophenyl, 2-thienyl, 2-pyridyl, 2-chloropyridyl or 5-bromopyridyl.

7. The fused ring compound with a sulfur-containing substituent according to claim 6, wherein,
R₁ is selected from methyl; R is selected from cyclopropyl or trifluoromethyl.

8. A method for preparing the fused ring compound with a sulfur-containing substituent of formula (I-a) according to claim 6, comprising: 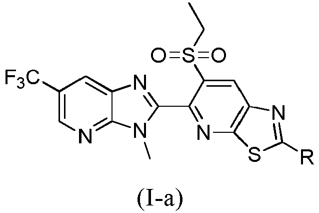
(1) stirring and refluxing a compound prepared by reacting a compound of formula (II) with a compound of formula (III) in acetic acid to give a compound of formula (IV);
(2) contacting the compound of formula (IV) with ethanethiol under an alkaline condition to give a compound of formula (V);
(3) reacting the compound of formula (V) with an oxidant to give a compound of formula (VI);
(4) conducting a Buchwald coupling reaction of the compound of formula (VI) and 2,4-dimethoxybenzylamine to give a compound of formula (VII);
(5) reacting the compound of formula (VII) with trifluoroacetic acid to give a compound of formula (VIII);
(6) reacting the compound of formula (VIII) with a brominating reagent to give a compound of formula (IX);
(7) reacting the compound of formula (IX) with acyl chloride in the presence of an acid binding agent to give a compound of formula (X);
(8) reacting the compound of formula (X) with a Lawson's reagent to give a compound of formula (XI);
(9) reacting the compound of formula (XI) in the presence of a base to give the final target compound of formula (I-a);

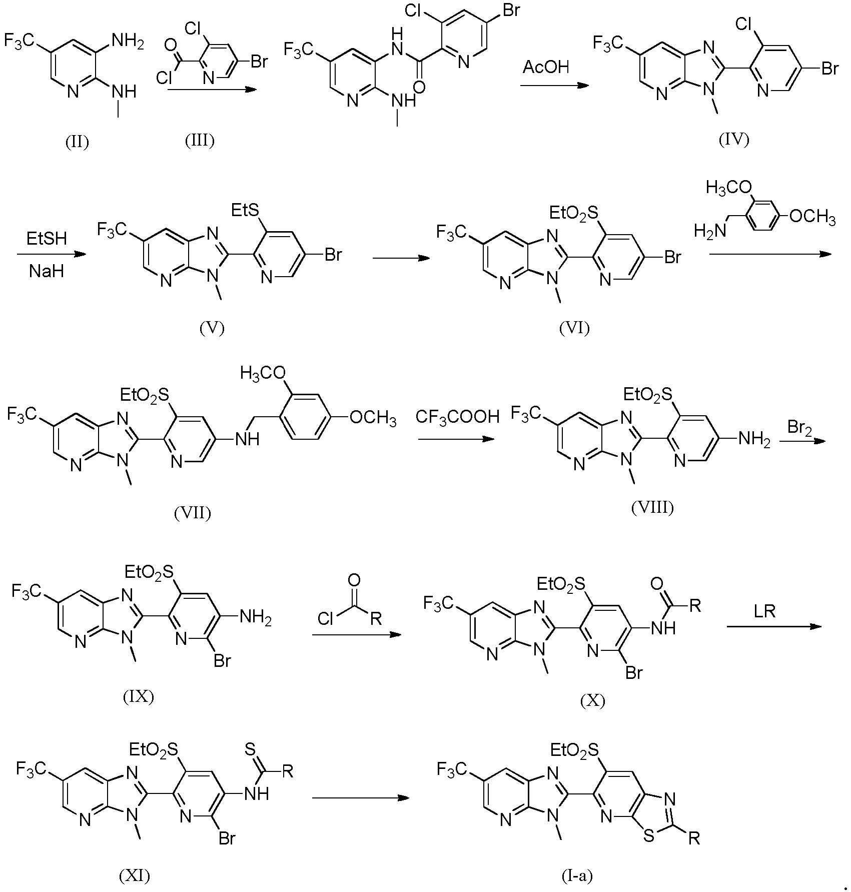

9. A method for preparing the fused ring compound with a sulfur-containing substituent of formula (I-b) according to claim 6, comprising: 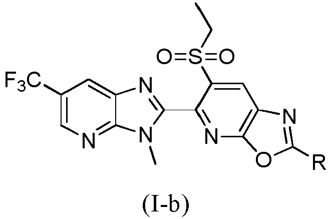 refluxing and stirring a compound of formula (X) in a solvent in the presence of CuI, a base and a catalyst to give the compound of formula (I-b);

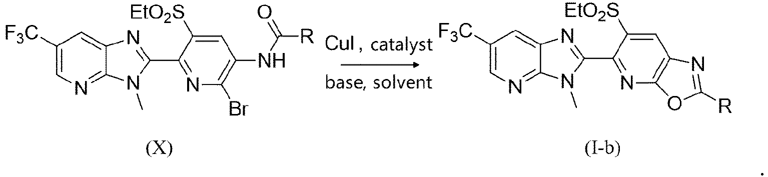

10. A method for preparing the fused ring compound with a sulfur-containing substituent of formula (I-c) according to claim 6, comprising: 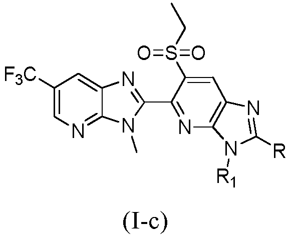
(1) refluxing and stirring a compound of formula (II) in acetic acid to give a compound of formula (III);
(2) reacting the compound of formula (III) with ethanethiol in the presence of a base to give a compound of formula (IV);
(3) oxidizing the compound of formula (IV) to give a compound of formula (V);
(4) contacting the compound of formula (V) with a substituted amine in a solvent to give a compound of formula (VI);
(5) dissolving the compound of formula (VI) in concentrated sulfuric acid, and then adding dilute nitric acid dropwise at low temperature to give a compound of formula (VII);
(6) reducing the compound of formula (VII) to give a compound of formula (VIII);
(7) reacting the compound of formula (VIII) with a substituted acyl chloride to give a compound of formula (IX);
(8) refluxing and stirring the compound of formula (IX) in acetic acid to give the compound of formula (I-c);

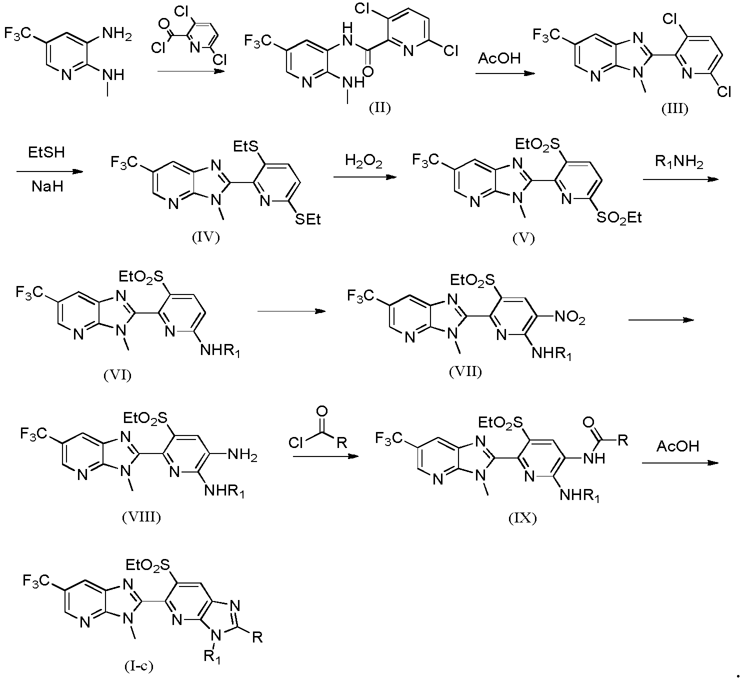

11. A method for preparing the fused ring compound with a sulfur-containing substituent of formula (I-d) according to claim 6, comprising: 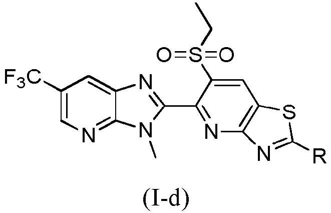
(1) contacting a compound of formula (V) obtained in (I-c) with ammonia solution in a solvent to give a compound of formula (X);
(2) brominating the compound of formula (X) in a solvent in the presence of a base to give a compound of formula (XI);
(3) reacting the compound of formula (XI) with a substituted acyl chloride to give a compound of formula (XII);
(4) reacting the compound of formula (XII) with a Lawson's reagent to give a compound of formula (XIII);
(5) conducting a cyclization reaction of the compound of formula (XIII) in the presence of a base to give the compound of formula (I-d);

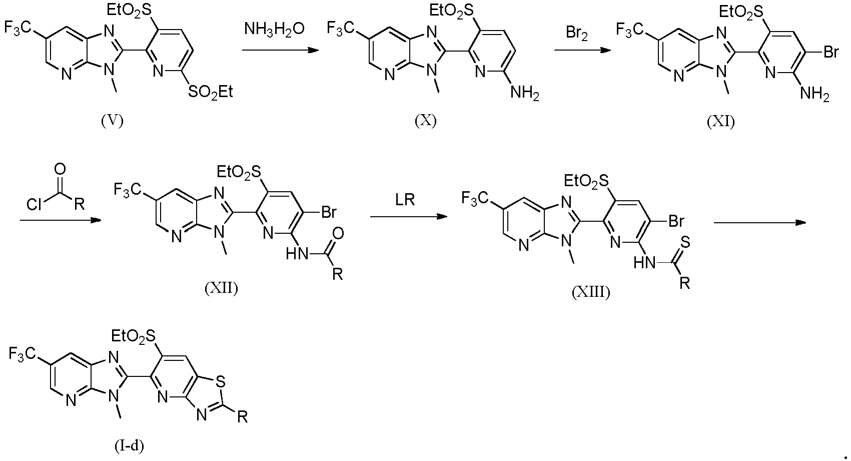

12. Use of a fused ring compound with a sulfur-containing substituent of formula (I) in the preparation of an insecticide.

13. The use according to claim 12, wherein the insecticide is an insecticide for controlling *lepidoptera* and/or *coleoptera.*

14. The use according to claim 12, wherein a pest targeted by the insecticide is selected from one or more of *nilaparvata lugens, laodelphax striatellus, bemisia tabaci, plutella xylostella, mythimna separata, myzus persicae,* and *tetranychus cinnabarinus.*

15. An insecticide, comprising the fused ring compound with a sulfur-containing substituent according to anyone of claims 1-6 and an adjuvant, wherein the concentration of the fused ring compound with a sulfur-containing substituent in the insecticide is 1-600ppm.
